# EUROPEAN PATENT SPECIFICATION

(11) **EP 4 267 007 B1**
(45) Date of publication and mention of the grant of the patent: **20.11.2024**
(21) Application number: 21819998.2
(22) Date of filing: 02.12.2021
(51) Int. Cl.: A61B 10/00, A61B 10/02, A61B 1/00

(54) **AN ENDOLUMINAL BIOPSY TOOL**
ENDOLUMINALES BIOPSIEWERKZEUG
OUTIL DE BIOPSIE ENDOLUMINALE

(30) Priority: 22.12.2020 NL 2027205
(43) Date of publication of application: 01.11.2023
(73) Proprietor: Technische Universiteit Delft, 2628 CN Delft (NL); Universitair Medisch Centrum Utrecht, 3584 CX Utrecht (NL)
(72) Inventor: SAKES, Aimée, 2600 AA Delft (NL); BREEDVELD, Paulus, 2600 AA Delft (NL); EUWE, Froukje Ellen, Utrecht (NL)
(74) Representative: van Breda, Jacobus
(86) International application number: PCT/NL2021/050731
(87) International publication number: WO 2022/139575

(56) References cited:
- CN-A- 106 166 079
- US-A1- 2010 094 168
- US-A1- 2012 245 487
- US-A1- 2013 053 726
- US-A1- 2016 120 524

## Description

The invention relates to an endoluminal biopsy tool, which is amongst other applications particularly suitable for use in mammary ductoscopy, said tool comprising an outer tube, and an instrument provided in the outer tube, wherein the outer tube and the instrument are movable with respect to each other for taking the biopsy. The term `endoluminal' with reference to the 'biopsy tool' expresses that the biopsy tool to which the invention relates has very limited cross-sectional dimensions which makes it suitable for use in regions of a patient's body wherein the tool must navigate through very narrow ducts or channels, such as in ductoscopy. The invention should however not be understood as being limited to a biopsy tool for use only in ductoscopy. Possible other applications are
- Lacrimal duct endoscopy
- Salivary duct endoscopy
- Pancreatic duct endoscopy
- Eustachian tube endoscopy
- Nephron duct endoscopy
- Bile duct endoscopy.

For the sake of clarity it is nevertheless considered expedient that the following description concentrates on the application of the endoluminal biopsy tool of the invention in ductoscopy.

Early detection of breast cancer plays a vital role in increasing the survival rate of women. Current breast cancer diagnosis modalities, such as mammography and echography, play an important role in the primary screening for breast cancer. MRI plays a vital role in primary screening to diagnose lesions, treatment selection, progression monitoring, and in determining cancer recurrence. However, by the time a lump is felt by the patient herself or with currently clinically available diagnostic tools, the lesion has been growing for approximately 8 years and is usually between Ø5-10 mm in size.

A minimally invasive micro-endoscopic technique called mammary ductoscopy is expected to allow for early breast cancer detection. Mammary ductoscopy allows for the visualization of some of the earliest lesions in situ (Ø0.1 mm) long before traditional imaging modalities, such as mammography, ultrasound, and MRI would allow detection. In mammary ductoscopy, a submillimetre fibreoptic micro-endoscope is inserted through one of the ductal openings onto the nipple surface of the breast to get direct access to the ductal epithelium.

US2006/0058703 discloses a tool usable in mammary ductoscopy, which tool comprises in accordance with the preamble (a) a substantially cylindrical cannula with a proximal end and a distal end, said cannula having at least one lateral opening, and (b) an endoscope (in connection with the intended application also referred to as ductoscope) which is axially movable inside the cannula. The at least one lateral opening of the cannula has at least in part a cutting region at its area being directed towards the distal end and/or at its area being directed towards the proximal end. In use a tissue sample is brought through the lateral opening into an interior of the cannula, and the tissue sample is separated from the rest of the tissue by moving the endoscope forward across the lateral opening and/or by retracting the endoscope, until the lateral opening is closed. In another embodiment of taking a biopsy the tissue sample is brought through the lateral opening into an interior of the cannula, and the tissue sample is separated from the rest of the tissue by moving the cannula with the lateral opening together with the fixed endoscope forward or backward, thereby manually exerting a gentle pressure against the tissue sample.

A major disadvantage of the tool known from US2006/0058703 is that taking the biopsy requires moving the endoscope or as it is called in this application the 'ductoscope', which implies that at the supreme moment of taking the biopsy the sight at actually taking the biopsy is lost.

An endoluminal biopsy tool according to the preamble of claim 1 is known from US 2010/094168 A1.

It is therefore an object of the invention to provide such a tool which is amongst others usable in mammary ductoscopy, and which provides the possibility to maintain sight during the process of actually taking the biopsy.

According to the invention an endoluminal biopsy tool is therefore proposed with the features of one or more of the appended claims.

In a first aspect and embodiment of the endoluminal biopsy tool of the invention, which tool comprises an outer tube and an instrument movably provided in the outer tube, the outer tube is provided with a tongue extending from a distal portion of the outer tube, and the instrument is an inner tube movably fitting inside the outer tube, wherein the inner tube has a wall that is provided with a cut out which is closable with the tongue.

There are several options that the tongue can close off the cut out. The cut out can for instance be closable by a translational advancing movement of the outer tube with respect to the inner tube.

By the advancing movement of the outer tube with respect to the inner tube the biopsy is taken from the tissue of interest. In that situation it is preferred that the tongue has a forward cutting edge.

Another option is that the cut out is closable by a rotational movement of the outer tube and the inner tube with respect to each other. Then with the rotational movement the biopsy is taken from the tissue of interest. The ease of taking the biopsy is then promoted by arranging that the tongue has cutting side edges.

In a second aspect and embodiment of the invention the outer tube and the inner tube are translational and/or rotationally movable with respect to each other, the inner tube is provided with a bevelled distal portion, and the tongue is provided with a preferential position wherein the tongue closes off the bevelled distal portion of the inner tube when the inner tube is placed in a partially retracted position with reference to the outer tube. The benefit of this feature is that by rotating or moving the outer tube forward and keeping the inner tube stationary, the tongue can be brought up to the point that it closes off the bevelled distal portion of the inner tube. During the rotational or forward movement of the tongue it can separate the biopsy from the tissue of interest and assist in moving the biopsy into the inner tube.

The operation of the tongue for closing off the distal portion of the inner tube and for taking the biopsy, is supported by the feature that the tongue has a preferential position wherein the tongue protrudes obliquely towards the inner tube when the tongue is unloaded.

Suitably therefore the tongue is resiliently mounted in an oblique position on the distal portion of the outer tube.

The first embodiment and the second embodiment can be applied independently and separately from each other. When the first embodiment and the second embodiment are combined it is preferable that the cut out is closable with the tongue when the inner tube extends beyond the distal portion of the outer tube, wherein the tongue is loaded by and engages the inner tube.

Suitably when the tongue is loaded by the inner tube, the tongue assumes an essentially straight orientation with reference to the outer tube.

In one embodiment the outer tube is embodied with a further cut out, having dimensions that are substantially the same as the dimensions of the cut out in the inner tube. The further cut out of this embodiment can effectively be used for cutting loose the biopsy tissue, in particular when the further cut out has cutting side edges.

The further cut out can be realized in different ways. The further cut out can be similar in shape to the cut out provided in the inner tube. It is however also possible that the further cut out extends through and is open at the distal portion of the outer tube.

The invention will hereinafter be further elucidated with reference to the drawing of an exemplary embodiment of an endoluminal biopsy tool according to the invention that is not limiting as to the appended claims. The exemplary embodiments refers to the application of the endoluminal biopsy tool in ductoscopy, but this is only for explanatory purposes without intention to restrict the scope of protection to the use of the endoluminal biopsy tool in ductoscopy.

In the drawing:
- figure 1 shows a schematic of current use of a ductoscope in conjunction with other features;
- figures 2, 3 and 4 show aspects of the endoluminal biopsy tool of the invention;
- figures 5 and 6 show another embodiment of the endoluminal biopsy tool of the invention; and
- figures 7 and 8 show still another embodiment of the endoluminal biopsy tool of the invention.

Whenever in the figures the same reference numerals are applied, these numerals refer to the same parts.

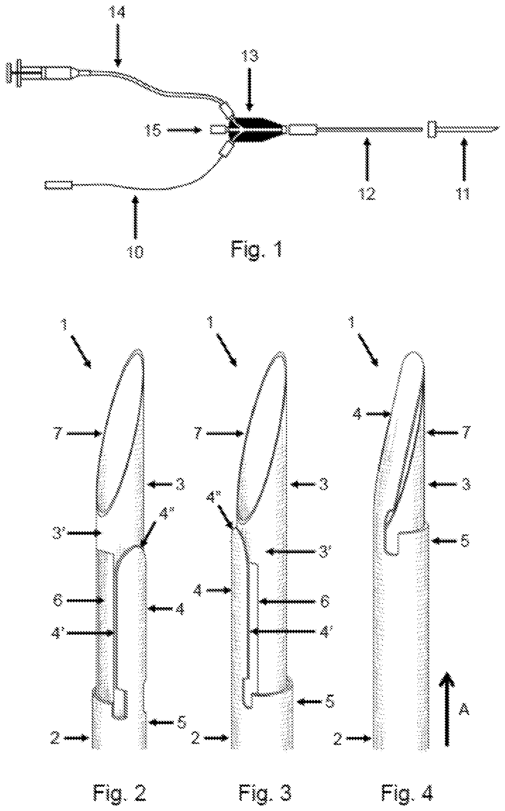

For a proper understanding of how the endoluminal biopsy tool may be used, figure 1 shows a schematic of current use of a ductoscope 10 in conjunction with a lumen expander 11, a cannula 12 and a specialized handle 13 that contains 2 or 3 lumens; one lumen for the ductoscope 10, one irrigation lumen 14 (in order to prevent the breast milk ducts from collapsing), and one lumen for an additional tool or treatment modality 15, such as insufflation, ductal lavage, and possible therapeutic interventions. The two main functions of the lumen expander 11 are 1) to provide access to the breast milk ducts via the nipple surface and 2) to extend the milk duct lumen. The cannula 12 provides a conduit for the ductoscope 10, a therapeutic tool 15, and irrigation 14. Irrigation is used to gently dilate the milk duct of interest using saline and allow for advancement of the ductoscope 10 into the duct until further advancement is no longer possible due to the size of the cannula (Ø1.15-1.4 mm). The lumen of the lumen expander 11 for the additional tool or treatment modality 15 is used for introduction of the endoluminal biopsy tool 1 of the invention into the breast under examination.

Figures 2, 3 and 4 show the endoluminal biopsy tool 1 of the invention. All figures show that the tool 1 comprises an outer tube 2 and an inner tube 3.

Figure 2 and figure 3 show aspects of a first embodiment of the biopsy tool 1 of the invention, wherein the outer tube 2 is provided with a tongue 4 extending from a distal portion 5 of the outer tube 2, and the inner tube 3 movably fits inside the outer tube 2. It is essential in this first embodiment that the inner tube 3 has a wall 3' that is provided with a cut out 6 which is closable with the tongue 4. The cut out 6 can for instance be closed by a translational advancing movement of the outer tube 2 with respect to the inner tube 3. For that purpose it is preferred that the tongue 4 has a forward cutting edge 4". It is also possible to close off the cut out 6 by a rotational movement of the outer tube 2 and the inner tube 3 with respect to each other. For that situation it is preferred that the tongue 4 has cutting side edges 4' .

Other features that are not necessarily present in the first embodiment and that relate to a second embodiment are also shown in figures 2 and 3. These other features will be referred to hereinafter predominantly with reference to figure 4.

In the second embodiment it is essential that the outer tube 2 and the inner tube 3 are translationally and/or rotationally movable with respect to each other. Figure 4 shows that in this second embodiment the inner tube 3 has a bevelled distal portion 7. The fact that this is also shown in figures 2 and 3 depicts that the first and the second embodiment may be combined.

Figure 4 further shows that the tongue 4 is provided with a preferential position wherein the tongue 4 closes off the bevelled distal portion 7 of the inner tube 3 when the inner tube 3 is placed in a partially retracted position with reference to the outer tube 2. This can be effectuated by a rotational movement of the outer tube 2 and the inner tube 3 with reference to each other, or by moving the outer tube 2 forward (as symbolized by arrow A) and keeping the inner tube 3 stationary. The latter movement causes that the tongue 4 likewise moves forward up to the point that it can close off the bevelled distal portion 7 of the inner tube 3. This is the situation shown in figure 4. During the forward movement of the tongue 4 it can separate the biopsy from the tissue of interest and assist in moving the biopsy into the inner tube 3.

One thing and another is preferably realized in that the tongue is movable between two positions, one position being preferable wherein the tongue 4 protrudes obliquely towards the inner tube 3 when the tongue 4 is unloaded. This is shown in figure 4. This can be suitably arranged by the feature that the tongue 4 is resiliently mounted in an oblique position on the distal portion 5 of the outer tube 2.

The other position of the tongue 4 comes in play when the tongue 4 is loaded by and engages the inner tube 3, wherein the tongue 4 assumes an essentially straight orientation with reference to the outer tube 2. This is shown in figures 2 and 3. Since these figures also show features of the first embodiment, the loaded position of the tongue 4 can then be used, as the figures show, to close off the cut out 6 with the tongue 4. It is then required that the inner tube 3 extends beyond the distal portion 5 of the outer tube 2.

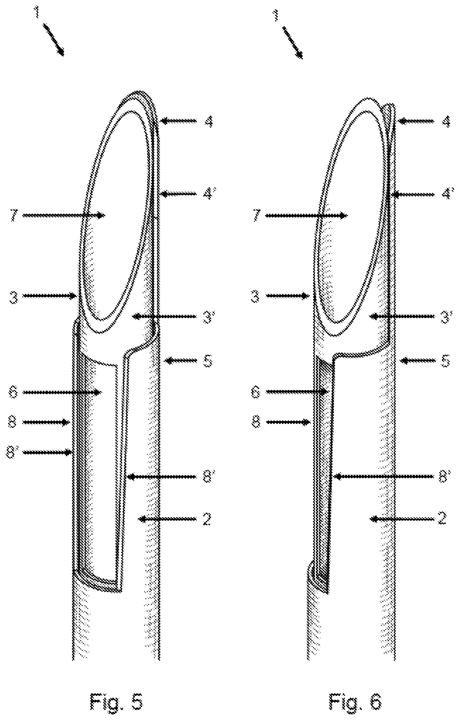

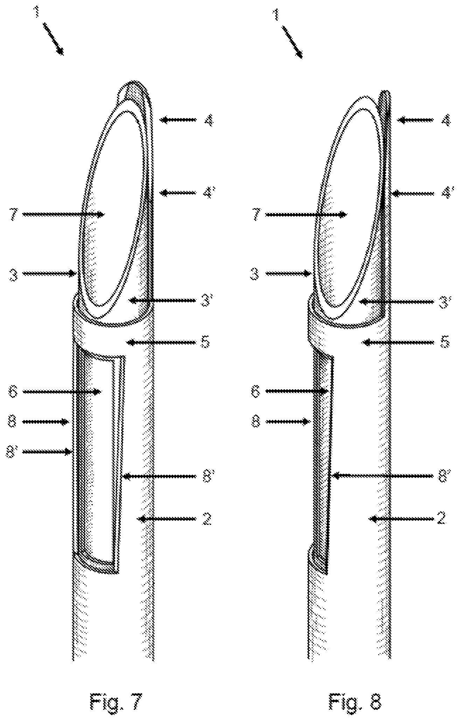

In the two embodiments shown in figures 5 and 6, and figures 7 and 8 respectively, in addition to the features discussed above with reference to the embodiment of figures 2, 3, and 4, the outer tube 2 is embodied with a further cut out 8, having dimensions that are substantially the same as the dimensions of the cut out 6 in the inner tube 3. This further cut out 8 also has a cutting functionality for the biopsy tissue. This is beneficially achieved in that the further cut out 8 has cutting side edges 8'. Rotation of the outer tube 2 with reference to the inner tube 3 when the cut out 6 in the inner tube 3 and the further cut out 8 in the outer tube 2 are overlaying with each other, results in scissor like cutting of tissue that is received in the cut outs 6, 8. The way this operates is clear from comparing figure 5 with figure 6 and from comparing figure 7 with figure 8. The difference between the two embodiments shown in figures 5 and 6, and figures 7 and 8 respectively is that in figures 5 and 6 the further cut out 8 extends through and is open at the distal portion 5 of the outer tube 2. In the embodiment of figures 7 and 8 the distal portion 5 of the outer tube 2 is closed in itself.

Although the invention has been discussed in the foregoing with reference to an exemplary embodiment of the biopsy tool of the invention, the invention is not restricted to this particular embodiment which can be varied in many ways without departing from the invention as defined in the independent claim. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

## Claims

1. An endoluminal biopsy tool (1), said tool (1) comprising an outer tube (2), and an instrument provided in the outer tube (2), wherein the outer tube (2) and the instrument are movable with respect to each other for taking the biopsy,
wherein the instrument is an inner tube (3) movably fitting inside the outer tube (2), wherein the inner tube (3) has a wall (3') that is provided with a cut out (6),
**characterized in that** the outer tube (2) is provided with a tongue (4) extending from a distal portion (5) of the outer tube (2), and further **characterised in that** the cut out (6) in the wall (3') of the inner tube (3) is closable with the tongue (4).

2. The endoluminal biopsy tool (1) of claim 1, **characterized in that** the cut out (6) is closable by a translational advancing movement of the outer tube (2) with respect to the inner tube (3).

3. The endoluminal biopsy tool (1) of claim 1 or 2, **characterized in that** the tongue (4) has a forward cutting edge.

4. The endoluminal biopsy tool (1) of any one of claims 1 - 3, **characterized in that** the cut out (6) is closable by a rotational movement of the outer tube (2) and the inner tube (3) with respect to each other.

5. The endoluminal biopsy tool (1) of any one of claims 1 - 4, **characterized in that** the tongue (4) has cutting side edges (4').

6. The endoluminal biopsy tool according to any one of claims 1 - 5, **characterized in that** the outer tube (2) and the inner tube (3) are translationally and/or rotationally movable with respect to each other, and the inner tube (3) has a bevelled distal portion (7), and the tongue (4) is provided with a preferential position wherein the tongue (4) closes off the bevelled distal portion (7) of the inner tube (3) when the inner tube (3) is placed in a partially retracted position with reference to the outer tube (2).

7. The endoluminal biopsy tool of claim 6, **characterized in that** the tongue (4) has a preferential position wherein the tongue (4) protrudes obliquely towards the inner tube (3) when the tongue (4) is unloaded.

8. The endoluminal biopsy tool of any one of claims 1 - 7, **characterized in that** the tongue (4) is resiliently mounted in an oblique position on the distal portion (5) of the outer tube (2).

9. The endoluminal biopsy tool of any one of claims 1 - 8, **characterized in that** the cut out (6) is closable with the tongue (4) when the inner tube (3) extends beyond the distal portion (5) of the outer tube (2), wherein the tongue (4) is loaded by and engages the inner tube (3).

10. The endoluminal biopsy tool of claim 9, **characterized in that** when the tongue (4) is loaded by the inner tube (3), the tongue (4) assumes an essentially straight orientation with reference to the outer tube (2).

11. The endoluminal biopsy tool of any one of claims 1 - 10, **characterized in that** the outer tube (2) is embodied with a further cut out (8), having dimensions that are substantially the same as the dimensions of the cut out (6) in the inner tube (3).

12. The endoluminal biopsy tool of claim 11, **characterized in that** the further cut out (8) has cutting side edges (8').

13. The endoluminal biopsy tool of claim 11 or 12, **characterized in that** the further cut out (8) extends through and is open at the distal portion (5) of the outer tube (2).

## Patentansprüche

1. Endoluminalbiopsie-Werkzeug (1), wobei das Werkzeug (1) eine äußere Röhre (2) und ein in der äußeren Röhre (2) vorgesehenes Instrument umfasst, wobei die äußere Röhre (2) und das Instrument zur Biopsienahme in Bezug aufeinander beweglich sind,
wobei das Instrument eine innere Röhre (3) ist, die beweglich in die äußere Röhre (2) passt, wobei die innere Röhre (3) eine Wand (3') aufweist, die mit einem Ausschnitt (6) versehen ist,
**dadurch gekennzeichnet, dass** die äußere Röhre (2) mit einer Zunge (4) versehen ist, die sich von einem distalen Abschnitt (5) der äußeren Röhre (2) erstreckt, und ferner **dadurch gekennzeichnet, dass** der Ausschnitt (6) in der Wand (3') der inneren Röhre (3) mit der Zunge (4) verschließbar ist.

2. Endoluminalbiopsie-Werkzeug (1) nach Anspruch 1, **dadurch gekennzeichnet, dass** der Ausschnitt (6) durch eine translatorische Vorschubbewegung der äußeren Röhre (2) in Bezug auf die innere Röhre (3) verschließbar ist.

3. Endoluminalbiopsie-Werkzeug (1) nach Anspruch 1 oder 2, **dadurch gekennzeichnet, dass** die Zunge (4) eine nach vorne gerichtete Schneidkante aufweist.

4. Endoluminalbiopsie-Werkzeug (1) nach einem der Ansprüche 1 - 3, **dadurch gekennzeichnet, dass** der Ausschnitt (6) durch eine Drehbewegung der äußeren Röhre (2) und der inneren Röhre (3) in Bezug aufeinander verschließbar ist.

5. Endoluminalbiopsie-Werkzeug (1) nach einem der Ansprüche 1 - 4, **dadurch gekennzeichnet, dass** die Zunge (4) schneidende Seitenkanten (4') aufweist.

6. Endoluminalbiopsie-Werkzeug gemäß einem der Ansprüche 1 - 5, **dadurch gekennzeichnet, dass** die äußere Röhre (2) und die innere Röhre (3) translatorisch und/oder rotatorisch in Bezug aufeinander beweglich sind und die innere Röhre (3) einen abgeschrägten distalen Abschnitt (7) aufweist und die Zunge (4) mit einer Vorzugsposition versehen ist, in der die Zunge (4) den abgeschrägten distalen Abschnitt (7) der inneren Röhre (3) verschließt, wenn die innere Röhre (3) in einer teilweise zurückgezogenen Position in Bezug auf die äußere Röhre (2) angeordnet ist.

7. Endoluminalbiopsie-Werkzeug nach Anspruch 6, **dadurch gekennzeichnet, dass** die Zunge (4) eine Vorzugsposition aufweist, in der die Zunge (4) schräg in Richtung der inneren Röhre (3) vorsteht, wenn die Zunge (4) unbelastet ist.

8. Endoluminalbiopsie-Werkzeug nach einem der Ansprüche 1 - 7, **dadurch gekennzeichnet, dass** die Zunge (4) elastisch in einer schrägen Position am distalen Abschnitt (5) der äußeren Röhre (2) angebracht ist.

9. Endoluminalbiopsie-Werkzeug nach einem der Ansprüche 1 - 8, **dadurch gekennzeichnet, dass** der Ausschnitt (6) mit der Zunge (4) verschließbar ist, wenn sich die innere Röhre (3) über den distalen Abschnitt (5) der äußeren Röhre (2) hinaus erstreckt, wobei die Zunge (4) durch die innere Röhre (3) belastet wird und gegen diese drückt.

10. Endoluminalbiopsie-Werkzeug nach Anspruch 9, **dadurch gekennzeichnet, dass** die Zunge (4) eine im Wesentlichen gerade Ausrichtung in Bezug auf die äußere Röhre (2) einnimmt, wenn die Zunge (4) durch die innere Röhre (3) belastet wird.

11. Endoluminalbiopsie-Werkzeug nach einem der Ansprüche 1 - 10, **dadurch gekennzeichnet, dass** die äußere Röhre (2) mit einem weiteren Ausschnitt (8) mit Abmessungen, die im Wesentlichen die gleichen sind, wie die Abmessungen des Ausschnitts (6) in der inneren Röhre (3), verwirklicht ist.

12. Endoluminalbiopsie-Werkzeug nach Anspruch 11, **dadurch gekennzeichnet, dass** der weitere Ausschnitt (8) schneidende Seitenkanten (8') aufweist.

13. Endoluminalbiopsie-Werkzeug nach Anspruch 11 oder 12, **dadurch gekennzeichnet, dass** der weitere Ausschnitt (8) sich durch den distalen Abschnitt (5) der äußeren Röhre (2) erstreckt und an diesem offen ist.

## Revendications

1. Outil de biopsie endoluminale (1), ledit outil (1) comprenant un tube externe (2), et un instrument disposé dans le tube externe (2), dans lequel le tube externe (2) et l'instrument sont mobiles l'un par rapport à l'autre pour effectuer la biopsie, dans lequel l'instrument est un tube interne (3) s'adaptant de manière mobile à l'intérieur du tube externe (2), dans lequel le tube interne (3) présente une paroi (3') qui est munie d'une découpe (6),
**caractérisé en ce que** le tube externe (2) est muni d'une languette (4) s'étendant depuis une partie distale (5) du tube externe (2), et en outre **caractérisé en ce que** la découpe (6) dans la paroi (3') du tube interne (3) est refermable avec la languette (4).

2. Outil de biopsie endoluminale (1) selon la revendication 1, **caractérisé en ce que** la découpe (6) est refermable par un mouvement d'avance en translation du tube externe (2) par rapport au tube interne (3).

3. Outil de biopsie endoluminale (1) selon la revendication 1 ou 2, **caractérisé en ce que** la languette (4) présente un bord de coupe avant.

4. Outil de biopsie endoluminale (1) selon l'une quelconque des revendications 1 à 3, **caractérisé en ce que** la découpe (6) est refermable par un mouvement de rotation du tube externe (2) et du tube interne (3) l'un par rapport à l'autre.

5. Outil de biopsie endoluminale (1) selon l'une quelconque des revendications 1 à 4, **caractérisé en ce que** la languette (4) présente des bords latéraux de coupe (4').

6. Outil de biopsie endoluminale selon l'une quelconque des revendications 1 à 5, **caractérisé en ce que** le tube externe (2) et le tube interne (3) sont mobiles en translation et/ou en rotation l'un par rapport à l'autre, et le tube interne (3) présente une partie distale biseautée (7), et la languette (4) est munie d'une position préférentielle dans laquelle la languette (4) referme la partie distale biseautée (7) du tube interne (3) lorsque le tube interne (3) est placé dans une position partiellement rétractée par rapport au tube externe (2).

7. Outil de biopsie endoluminale selon la revendication 6, **caractérisé en ce que** la languette (4) présente une position préférentielle dans laquelle la languette (4) fait saillie de manière oblique vers le tube interne (3) lorsque la languette (4) est déchargée.

8. Outil de biopsie endoluminale selon l'une quelconque des revendications 1 à 7, **caractérisé en ce que** la languette (4) est montée de manière élastique dans une position oblique sur la partie distale (5) du tube externe (2).

9. Outil de biopsie endoluminale selon l'une quelconque des revendications 1 à 8, **caractérisé en ce que** la découpe (6) est refermable avec la languette (4) lorsque le tube interne (3) s'étend au-delà de la partie distale (5) du tube externe (2), dans lequel la languette (4) est chargée par et vient en prise avec le tube interne (3).

10. Outil de biopsie endoluminale selon la revendication 9, **caractérisé en ce que** la languette (4) est chargée par le tube interne (3), la languette (4) adopte une orientation sensiblement droite par rapport au tube externe (2).

11. Outil de biopsie endoluminale selon l'une quelconque des revendications 1 à 10, **caractérisé en ce que** le tube externe (2) est pourvu d'une autre découpe (8), présentant des dimensions qui sont sensiblement identiques aux dimensions de la découpe (6) dans le tube interne (3).

12. Outil de biopsie endoluminale selon la revendication 11, **caractérisé en ce que** l'autre découpe (8) présente des bords latéraux de coupe (8').

13. Outil de biopsie endoluminale selon la revendication 11 ou 12, **caractérisé en ce que** l'autre découpe (8) s'étend à travers et est ouverte au niveau de la partie distale (5) du tube externe (2).
